# EUROPÄISCHE PATENTSCHRIFT

(11) **EP 4 344 662 B1**
(45) Veröffentlichungstag und Bekanntmachung des Hinweises auf die Patenterteilung: **23.04.2025**
(21) Anmeldenummer: 22198067.5
(22) Anmeldetag: 27.09.2022
(51) Int. Cl.: A61B 18/00, G02B 6/00

(54) **VERFAHREN ZUR PRÜFUNG EINER AUSRICHTUNG EINES DISTALEN ENDES EINES LICHTWELLENLEITERS SOWIE PRÜFVORRICHTUNG ZUR DURCHFÜHRUNG DES VERFAHRENS**
METHOD FOR TESTING AN ALIGNMENT OF A DISTAL END OF AN OPTICAL WAVEGUIDE AND TESTING DEVICE FOR CARRYING OUT THE METHOD
PROCÉDÉ DE CONTRÔLE D'UNE ORIENTATION D'UNE EXTRÉMITÉ DISTALE D'UN GUIDE D'ONDES OPTIQUES ET DISPOSITIF DE CONTRÔLE POUR LA MISE EN OEUVRE DU PROCÉDÉ

(43) Veröffentlichungstag der Anmeldung: 03.04.2024
(73) Patentinhaber: Erbe Elektromedizin GmbH, 72072 Tübingen (DE)
(72) Erfinder: REITERER, Markus, 2620 Loipersbach (AT); DRAGOSTINOFF, Nikolaus, 1230 Wien (AT); HAHNEKAMP, Andreas, 7032 Sigleß (AT)
(74) Vertreter: Rüger Abel Patentanwälte PartGmbB

(56) Entgegenhaltungen:
- EP-A1- 3 508 899
- US-A1- 2011 268 141
- US-A1- 2017 167 861
- US-A1- 2021 236 187

## Beschreibung

Die Erfindung betrifft ein Verfahren zur Prüfung einer Ausrichtung und/oder Positionierung eines distalen Endes eines Lichtwellenleiters, der Bestandteil eines medizinischen Instruments ist. Das Verfahren ist dazu eingerichtet, zu prüfen, ob das distale Ende und insbesondere eine distale Stirnfläche am distalen Ende des Lichtwellenleiters gegenüber einem Griffstück und/oder einer Elektrode des medizinischen Instruments korrekt ausgerichtet und/oder positioniert ist. Die Erfindung betrifft außerdem eine Prüfvorrichtung, die zur Durchführung des Verfahrens eingerichtet ist.

Das Verfahren und die Prüfvorrichtung können beispielsweise zumindest stichprobenartig zur Prüfung von hergestellten medizinischen Instrumenten eingesetzt werden, um deren Funktionsfähigkeit bzw. ausreichende Qualität sicherzustellen. Sie können zusätzlich oder alternativ auch dazu verwendet werden, Mehrweginstrumente nach dem Gebrauch, der Reinigung oder der Sterilisation zu prüfen.

US 2017/0 167 861 A1 beschreibt ein Verfahren zur Überprüfung der Ausrichtung eines Lichtwellenleiters gegenüber einer Linse, insbesondere einer GRIN-Linse. Dabei wird Licht von der Faser durch die Linse auf eine Projektionsfläche abgestrahlt. Die Faser wird dann relativ zur Linse bewegt, bis die gewünschte Ausrichtung erreicht ist. Dies wird durch Zentrieren eines Leuchtflecks auf der Projektionsfläche geprüft bzw. erkannt.

Bei dem aus US 2011/0 228 259 A1 bekannten Verfahren wird die korrekte Anordnung eines oder mehrerer Lichtwellenleiter an einem damit verbundenen Kopplungselement mittels eines Sensors geprüft. Dazu wird Licht über den wenigstens einen Lichtwellenleiter und das Kopplungselement und einen Sensor abgestrahlt und dann festgestellt, ob das abgestrahlte Licht an der korrekten Position auf dem Sensor einfällt. Außerdem kann auch die Intensität des auf dem Sensor einfallenden Lichts geprüft werden, um die Transmission durch die Anordnung aus dem wenigstens einen Lichtwellenleiter und dem Kopplungselement zu prüfen. Ferner sei auf das Dokument US2021/0236187 A1 verwiesen.

Ausgehend von dem bekannten Stand der Technik kann es als Aufgabe der vorliegenden Erfindung angesehen werden, die Prüfung einer Ausrichtung eines distalen Endes eines Lichtwellenleiters eines medizinischen Instruments mit einfachen Mitteln zu verbessern.

Diese Aufgabe wird durch ein Verfahren mit den Merkmalen des Patentanspruches 1 sowie eine Prüfvorrichtung mit den Merkmalen des Patentanspruches 13 gelöst.

Erfindungsgemäß wird die Ausrichtung und/oder Positionierung des distalen Endes eines Lichtwellenleiters eines medizinischen Instruments relativ zu einem Griffstück und/oder relativ zu einer Elektrode des medizinischen Instruments geprüft, um dessen bestimmungsgemäße Funktionsweise sicherzustellen.

Der Lichtwellenleiter des medizinischen Instruments hat an einem distalen Ende eine distale Stirnfläche und an einem proximalen Ende eine proximale Stirnfläche. Während der bestimmungsgemäßen Verwendung des medizinischen Instruments, zum Beispiel bei einem elektrochirurgischen Eingriff, kann Licht an der distalen Endfläche empfangen und über den Lichtwellenleiter zum proximalen Ende geleitet. Dort wird das Licht an der proximalen Stirnfläche zumindest teilweise ausgekoppelt und kann ausgewertet werden, beispielsweise, um das mit dem medizinischen Instrument behandelte Gewebe zu bestimmen bzw. zu klassifizieren. Die Ausrichtung und/oder Positionierung des distalen Endes des Lichtwellenleiters ist von Bedeutung, damit ausreichend Licht empfangen werden kann.

Der Lichtwellenleiter kann optional wenigstens eine Ummantelungsschicht aufweisen, die den Kern des Lichtwellenleiters umgibt. Als proximale und distale Stirnfläche des Lichtwellenleiters jeweils die Stirnfläche des Kerns des Lichtwellenleiters ohne die wenigstens eine Ummantelungsschicht angesehen.

Während des erfindungsgemäßen Verfahrens wird Licht zur Prüfung der Ausrichtung des distalen Endes durch den Lichtwellenleiter geleitet. Es wird mittels einer Lichtquelle in die proximale Stirnfläche eingekoppelt. Das Einkoppeln des Lichts an der proximalen Stirnfläche erfolgt in einem ersten Prüfzustand unsymmetrisch und/oder exzentrisch relativ zu einem Mittelpunkt der proximalen Stirnfläche. Die proximale Stirnfläche wird daher nicht gleichmäßig mit Licht bestrahlt. Vorzugsweise wird nur in einen zusammenhängenden oder nicht zusammenhängenden Flächenbereich der proximalen Stirnfläche Licht eingekoppelt, der nicht rotationssymmetrisch gegenüber dem Mittelpunkt der proximalen Stirnfläche ist oder dessen geometrischer Schwerpunkt versetzt zum Mittelpunkt der proximalen Stirnfläche angeordnet ist. Insbesondere wird die proximale Stirnfläche nicht vollflächig kreisförmig konzentrisch zu ihrem Mittelpunkt mit Licht bestrahlt. Dieser erste Prüfzustand wird verwendet, um die Relativposition und Ausrichtung des distalen Endes des Lichtwellenleiters relativ zum Griffstück und/oder der Elektrode zu prüfen.

Das an der proximalen Stirnfläche eingekoppelte Licht wird zumindest teilweise an der distalen Stirnfläche auf eine Projektionsfläche abgestrahlt und erzeugt auf der Projektionsfläche ein Lichtmuster. Beispielsweise handelt es sich bei dem Lichtmuster um einen geschlossenen Ring oder einen Ringbogen. Die distale Stirnfläche ist dabei gegenüber der Projektionsfläche in einer definierten Position bzw. Ausrichtung. Dies hat zur Folge, dass auch die Elektrode bzw. das Griffstück des medizinischen Instruments gegenüber der Projektionsfläche eine definierte Position und/oder Ausrichtung hat. Anhand des auf der Projektionsfläche erzeugten Lichtmusters kann daher die Relativposition und Ausrichtung des distalen Endes relativ zum Griffstück und/oder der Elektrode beurteilt werden. Hierzu wird das erzeugte Lichtmuster anhand wenigstens eines vorgegebenen Prüfkriteriums geprüft. Beispielsweise können ein oder mehrere der folgenden Prüfkriterien verwendet werden:
- Vergleich der Istform oder Istgeometrie des Lichtmusters mit einer Sollform oder Sollgeometrie;
- Vergleich eines Istwertes wenigstens einer Dimension parallel zur Projektionsfläche mit jeweils einem zugeordneten Sollwert.

Bei dem Vergleich von Sollwerten mit Istwerten kann das Prüfkriterium eine zulässige Abweichung zwischen dem jeweiligen Sollwert und dem jeweiligen Istwert vorgeben.

Ergibt die Auswertung, dass das Lichtmuster das wenigstens eine Prüfkriterium erfüllt, ist das distale Ende des Lichtwellenleiters korrekt positioniert und/oder ausgerichtet. Andernfalls entspricht das medizinische Instrument nicht den Vorgaben und kann als Folge davon z.B. instand gesetzt und/oder aussortiert werden.

Das Verfahren kann im Rahmen der Produktion von medizinischen Instrumenten stichprobenweise für einen Teil der hergestellten medizinischen Instrumente oder für alle hergestellten medizinischen Instrumente durchgeführt werden.

Es ist auch möglich, ein medizinisches Instrument nach einer oder mehreren Verwendungen jeweils zu prüfen, um Veränderungen in der Relativposition bzw. der Relativausrichtung des distalen Endes des Lichtwellenleiters gegenüber dem Griffstück und/oder der Elektrode festzustellen. Solche Veränderungen können beispielsweise durch Verformungen und/oder plastische Veränderungen an Verbindungsstellen, insbesondere Klebestellen, auftreten, beispielsweise infolge der Sterilisation des medizinischen Instruments.

Optional kann mittels des erfindungsgemäßen Verfahrens und/oder der erfindungsgemäßen Prüfvorrichtung zwischen dem ersten Prüfzustand und einem weiteren, zweiten Prüfzustand umgeschaltet werden. Dieser optionale zweite Prüfzustand dient dazu, den Sitz der Elektrode im oder am Griffstück bzw. die Relativposition zwischen der Elektrode und dem Griffstück zu prüfen. Dazu wird am distalen Ende des Lichtwellenleiters Licht derart abgestrahlt, dass sich die Elektrode im Lichtweg zwischen der distalen Stirnfläche und der Projektionsfläche befindet. Dadurch ist auf der Projektionsfläche ein Schattenbild der Elektrode zu erkennen. Die Position und/oder Ausrichtung des Schattenbilds relativ zum Lichtmuster auf der Projektionsfläche kann ausgewertet werden, um die Relativposition zwischen der Elektrode einerseits und dem Griffstück und dem Lichtwellenleiter andererseits zu prüfen. Es kann somit geprüft werden, ob die Elektrode korrekt am Griffstück angeordnet ist.

Im zweiten Prüfzustand kann auf der Projektionsfläche ein zusammenhängendes, vorzugsweise kreisförmiges, flächiges Lichtmuster erzeugt werden. Im zweiten Prüfzustand und ohne Berücksichtigung des Schattenbildes der Elektrode bildet das Lichtmuster auf der Projektionsfläche insbesondere eine vollständig ausgeleuchtete, vorzugsweise kreisförmige Lichtfläche. Im zweiten Prüfzustand kann das Licht der Lichtquelle symmetrisch und/oder konzentrisch und/oder in Form eines zusammenhängenden, vorzugsweise kreisförmigen, vollflächigen Lichtflecks relativ zu einem Mittelpunkt der proximalen Stirnfläche an der proximalen Stirnfläche des Lichtwellenleiters eingekoppelt werden. Zusätzlich oder alternativ kann der Lichtwellenleiter im zweiten Prüfzustand zwischen seinem distalen Ende und seinem proximalen Ende derart angeordnet sein, dass eine Modenmischung auftritt.

Bei dem medizinischen Instrument handelt es sich vorzugsweise um ein monopolares oder bipolares elektrochirurgisches Instrument, das insbesondere zur Koagulation und/oder Dissektion von menschlichem und/oder tierischem Gewebe eines Patienten verwendet wird.

Wie bereits erwähnt, kann das Prüfkriterium oder eines der verwendeten Prüfkriterien das Prüfen der Abweichung zwischen einer Istform und einer Sollform des Lichtmusters sein. Dabei kann die Istform und die Sollform beispielsweise ein Kreisring oder ein Kreisringbogen sein. Die Istform kann beispielsweise dahingehend geprüft werden, ob sie die gleiche Rundheit aufweist wie die Sollform oder ähnliches. Zusätzlich oder alternativ kann beispielsweise der Istwert eines Innendurchmessers und/oder der Istwert eines Außendurchmessers des Kreisrings oder des Kreisringbogens mit jeweils einem zugeordneten Sollwert verglichen werden. Generell können eine oder mehrere Istwerte einer Dimension mit einem jeweils zugeordneten Sollwert für diese Dimension verglichen werden, beispielsweise auch die Breite bzw. Dicke eines Kreisrings oder Kreisringbogens (Differenz zwischen dem Außendurchmesser und dem Innendurchmesser).

Vorzugsweise hat das in die proximale Stirnfläche eingekoppelte Licht ein Spektrum, das auch eine oder mehrere der Lichtwellenlängen enthält, die bei der bestimmungsgemäßen Verwendung auftreten können, z.B. während eines elektrochirurgischen Eingriffs an wenigstens einem Gewebetyp.

Die Auswertung des Lichtmusters kann automatisch oder durch eine Bedienperson erfolgen. In beiden Fällen können auf der Projektionsfläche Markierungen vorhanden sein, die das Anwenden des wenigstens einen Prüfkriteriums erleichtern und beispielsweise einen Sollwert für eine Dimension und/oder eine Sollform für das Lichtmuster angeben.

Bei einer bevorzugten Ausführungsform wird das Lichtmuster auf der Projektionsfläche mittels einer Kamera erfasst. Die Projektionsfläche kann an einem zumindest teilweise transparenten Schirm vorhanden sein, so dass die Kamera das erzeugte Lichtmuster, auf der der Projektionsfläche entgegengesetzten Rückfläche des Schirms erfassen kann. Die Kamera kann auch auf die Projektionsfläche gerichtet sein. Vorzugsweise ist die optische Achse der Kamera rechtwinklig zur Projektionsfläche angeordnet. Die Projektionsfläche ist insbesondere eine ebene Fläche.

Bei einer bevorzugten Ausführungsform ist der Lichtwellenleiter eine Multimodenfaser (MMF). In diesem Fall ist es vorteilhaft, wenn der Lichtwellenleiter zwischen seinem distalen Ende und seinem proximalen Ende derart angeordnet ist, dass eine Modenmischung vermieden wird. Hierzu wird der Lichtwellenleiter zwischen dem distalen Ende und dem proximalen Ende ausreichend gestreckt bzw. ausreichend wenig gekrümmt angeordnet. Vorzugsweise bildet er zwischen dem proximalen Ende und dem distalen Ende keine derart gekrümmten Bögen und/oder Schleifen, die eine Modenmischung verursachen würden. Beispielsweise kann sich der Lichtwellenleiter zwischen dem proximalen Ende und dem distalen Ende geradlinig erstrecken.

Die proximale Stirnfläche und/oder die distale Stirnfläche kann bzw. können eben oder konvex sein. In beiden Fällen kann eine betreffende Stirnfläche eine polierte Fläche sein. Eine ebene Stirnfläche kann auch eine nicht nachbearbeitete Bruchfläche sein.

Bei einer bevorzugten Ausführungsform wird mittels der Lichtquelle ein zusammenhängender Lichtfleck in einem Flächenbereich der proximalen Stirnfläche erzeugt, der exzentrisch gegenüber dem Mittelpunkt der proximalen Stirnfläche angeordnet ist. Der Flächenschwerpunkt des Lichtflecks bzw. Flächenbereichs ist dabei versetzt zum Mittelpunkt der proximalen Stirnfläche angeordnet. Der Lichtfleck bzw. Flächenbereich kann beispielsweise kreisförmig oder elliptisch sein. Alternativ dazu kann der Flächenbereich auch durch mehrere separate Abschnitte, beispielsweise mehrere separate kreisförmige und/oder elliptische Lichtflecken gebildet sein. Bei einem weiteren Ausführungsbeispiel kann der Flächenbereich auch ringförmig, konzentrisch oder exzentrisch zum Mittelpunkt der proximalen Stirnfläche angeordnet sein. In jedem Fall ist der Flächenbereich, in dem Licht in den Lichtwellenleiter eingekoppelt wird, radial zum Mittelpunkt der proximalen Stirnfläche inhomogen und/oder in Umfangsrichtung um den Mittelpunkt der proximalen Stirnfläche inhomogen.

Die erfindungsgemäße Prüfvorrichtung weist eine Lichtquelle, eine Projektionsfläche sowie eine Halterung auf. Die Halterung ist dazu eingerichtet, dass ein Bestandteil des medizinischen Instruments, der mit dem distalen Ende des Lichtwellenleiters verbunden ist, in einer vorgegebenen Relativposition bzw. Relativausrichtung relativ zur Projektionsfläche angeordnet wird. Die Lichtquelle ist dazu eingerichtet, Licht in die proximale Stirnfläche unsymmetrisch und/oder exzentrisch einzukoppeln. Die Prüfvorrichtung ist dazu eingerichtet, irgendein Ausführungsbeispiel des vorstehend beschriebenen Verfahrens durchführen zu können.

Die Auswertung des Lichtmusters kann automatisch oder durch eine Bedienperson erfolgen. Zur automatischen Auswertung kann die Prüfvorrichtung optional eine Kamera aufweisen. Wenigstens ein von der Kamera erfasstes Bild des Lichtmusters kann an eine Zentraleinheit bzw. Auswerteeinheit übermittelt werden. Die Zentraleinheit ist in diesem Fall dazu eingerichtet, das wenigstens eine Bild des Lichtmusters automatisch basierend auf dem wenigstens einen Prüfkriterium auszuwerten. Die Zentraleinheit kann optional dazu eingerichtet sein, die Kamera und/oder die Lichtquelle zu steuern.

Vorteilhafte Ausgestaltungen der Erfindung ergeben sich aus den abhängigen Ansprüchen, der Beschreibung und der Zeichnung. Nachfolgend werden bevorzugte Ausführungsbeispiele der Erfindung anhand der beigefügten Zeichnungen im Einzelnen erläutert. In der Zeichnung zeigen:
Figur 1 eine schematische Darstellung eines Ausführungsbeispiels eines medizinischen Instruments sowie eines Geräts, an das das medizinische Instrument während des Betriebs angeschlossen ist,
Figur 2 ein Blockschaltbild eines Ausführungsbeispiels einer Prüfvorrichtung für das medizinische Instrument zur Prüfung der Position und/oder Ausrichtung eines Lichtwellenleiters relativ zu einem Griffstück bzw. einer Elektrode des medizinischen Instruments,
Figur 3 eine schematische Prinzipdarstellung einer distalen Stirnfläche des Lichtwellenleiters des medizinischen Instruments aus Figuren 1 und 2 sowie eines Flächenbereichs, in dem Licht in die proximale Stirnfläche des Lichtwellenleiters in einem ersten Prüfzustand eingekoppelt wird und
Figur 4 eine schematische Prinzipdarstellung eines auf einer Projektionsfläche der Prüfvorrichtung erzeugten Lichtmusters im ersten Prüfzustand,
Figur 5 ein schematische Prinzipdarstellung der distalen Stirnfläche des Lichtwellenleiters des medizinischen Instruments aus Figuren 1 bis 3 sowie eines Flächenbereichs, in dem Licht in die proximale Stirnfläche des Lichtwellenleiters in einem zweiten Prüfzustand eingekoppelt wird und
Figur 6 eine schematische Prinzipdarstellung eines auf einer Projektionsfläche der Prüfvorrichtung erzeugten Lichtmusters im zweiten Prüfzustand.

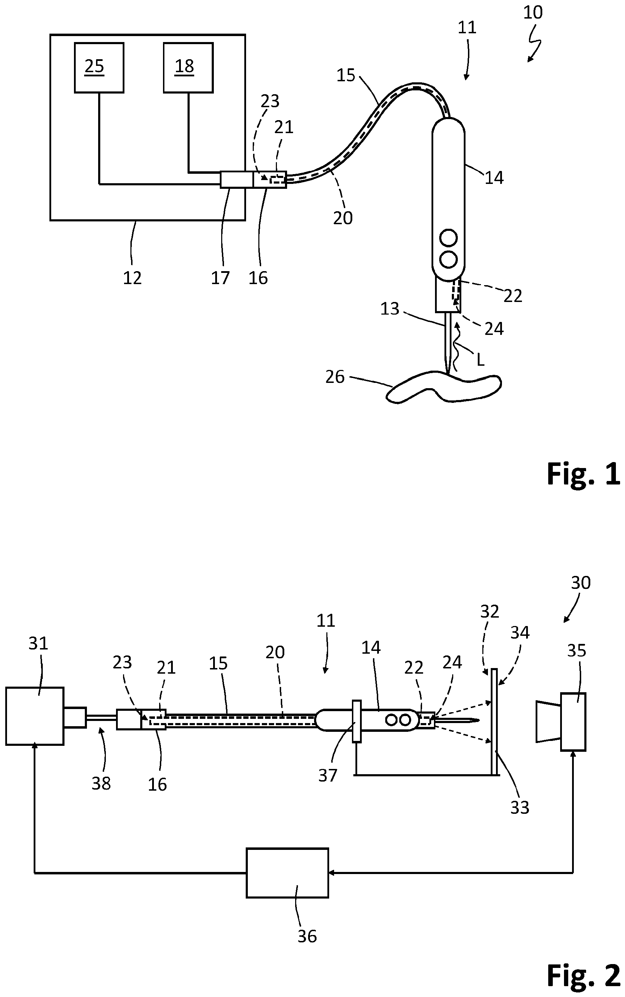

Figur 1 zeigt ein System 10, aufweisend ein medizinisches Instrument 11 sowie ein Gerät 12, an das das medizinische Instrument 11 anschließbar ist. Das medizinische Instrument 11 ist ein elektrochirurgisches Instrument und kann als monopolares oder bipolares medizinisches Instrument 11 mit wenigstens einer Elektrode 13 ausgebildet sein. Die Elektrode 13 ist an einem Griffstück 14 des medizinischen Instruments 11 angeordnet.

Ein Kabel 15 führt vom Griffstück 14 mit der Elektrode 13 zu einem Verbindungselement 16. Am Gerät 12 ist ein Anschluss 17 vorhanden, an den das Verbindungselement 16 angeschlossen werden kann. Beispielsgemäß wird mittels des Verbindungselements 16 und dem Anschluss 17 zumindest eine elektrische sowie eine optische Verbindung zwischen dem Gerät 12 und dem medizinischen Instrument 11 hergestellt.

Die Elektrode 13 ist bei hergestellter Verbindung zwischen dem medizinischen Instrument 11 und dem Gerät 12 über wenigstens einen elektrischen Leiter mit einer elektrischen Energiequelle 18 des Geräts 12 verbunden. Der wenigstens eine elektrische Leiter ist Bestandteil des Kabels 15 und in der Zeichnung nicht veranschaulicht.

Im Kabel 15 erstreckt sich außerdem ein Lichtwellenleiter 20 von einem proximalen Ende 21 am Verbindungselement 16 zu einem distalen Ende 22 am Griffstück 14. Das distale Ende 22 ist relativ zum Griffstück 14 unbeweglich am Griffstück 14 befestigt, vorzugsweise durch eine stoffschlüssige Verbindung, insbesondere eine Klebeverbindung. Zusätzlich oder alternativ kann das distale Ende 22 auch kraftschlüssig und/oder formschlüssig mit dem Griffstück 14 verbunden sein.

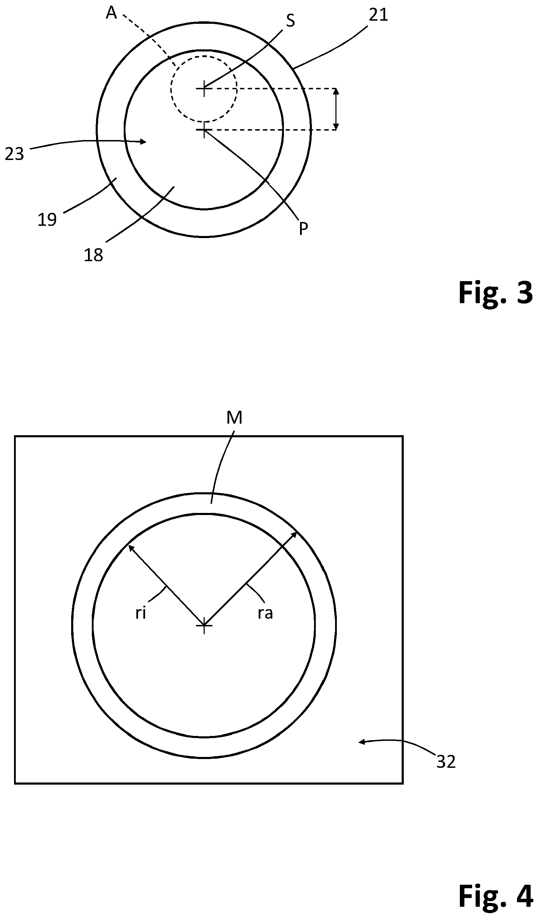

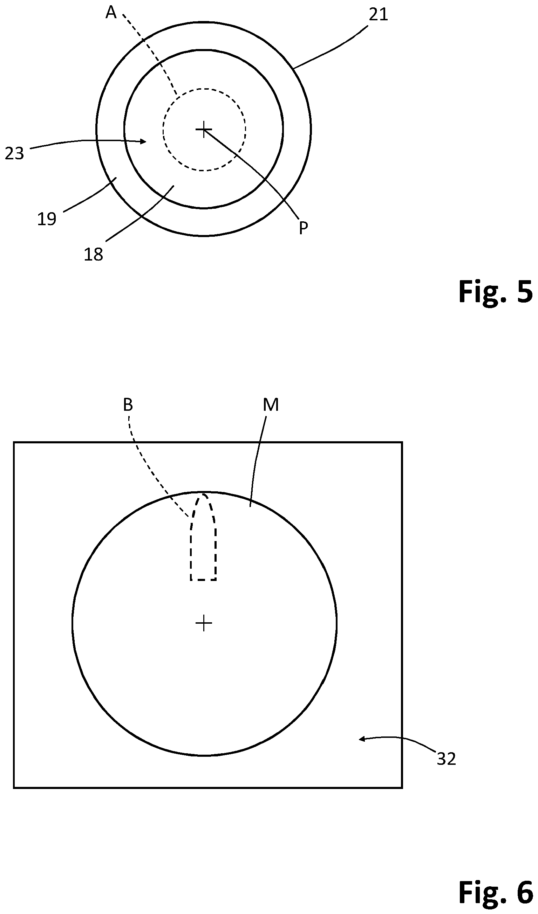

Am proximalen Ende 21 weist der Lichtquellenleiter 20 eine proximale Stirnfläche 23 auf und am distalen Ende 22 weist der Lichtwellenleiter 20 eine distale Stirnfläche 24 auf. Der Lichtwellenleiter 20 kann einen Kern 18 und eine Ummantelung 19 aufweisen (Figuren 3 und 5). Als proximale Stirnfläche 23 und distale Stirnfläche 24 werden beispielsgemäß die Stirnflächen des Kerns 18 ohne die Ummantelung 19 angesehen.

Bei hergestellter Verbindung wird über das Verbindungselement 16 und den Anschluss 17 eine optische Verbindung zwischen dem proximalen Ende 21 und einer Auswerteeinheit 25 des Geräts 12 hergestellt.

Das medizinische Instrument 11 ist beispielsgemäß als elektrochirurgisches Instrument ausgebildet, wobei mittels der wenigstens einen Elektrode 13 tierisches oder menschliches Gewebe 26 behandelt werden kann, beispielsweise mittels Koagulation und/oder Dissektion. Bei der Behandlung des Gewebes 26 erzeugtes Licht L wird an der distalen Stirnfläche 24 empfangen und über den Lichtwellenleiter 20 zum proximalen Ende 21 geleitet. Am proximalen Ende 21 wird das Licht L zumindest teilweise über den Anschluss 17 zur Auswerteeinheit 25 weitergeleitet und kann dort ausgewertet werden, beispielsweise um das Gewebe 26 zu klassifizieren und/oder zu bestimmen.

Um eine solche Bewertung des behandelten Gewebes 26 vornehmen zu können, muss der Lichtwellenleiter 20 und insbesondere dessen distales Ende 22 sehr exakt relativ zum Griffstück 14 bzw. relativ zur Elektrode 13 angeordnet und/oder ausgerichtet sein, um ausreichend Licht L aufzunehmen, das während der Behandlung des Gewebes 26 erzeugt wird. Ansonsten ist eine Bewertung oder Klassifizierung des Gewebes 26 nicht oder nur unzureichend möglich.

Erfindungsgemäß wird daher das medizinische Instrument 11 geprüft, ob die Ausrichtung und/oder Positionierung des distalen Endes 22 des Lichtwellenleiters 20 gegenüber dem Griffstück 14 und/oder der wenigstens einen Elektrode 13 korrekt ist und den Vorgaben entspricht. Dazu kann eine Prüfvorrichtung 30 verwendet werden, wie sie in Figur 2 schematisch veranschaulicht ist. Die Prüfvorrichtung 30 weist eine Lichtquelle 31 zur Einkopplung von Licht in die proximale Stirnfläche 23 des Lichtwellenleiters 20 sowie eine Projektionsfläche 32 auf. Die Projektionsfläche 32 ist so angeordnet und ausgerichtet, dass Licht, das aus der distalen Stirnfläche 24 des Lichtwellenleiters 20 austritt, auf die Projektionsfläche 32 auftrifft und dort ein Lichtmuster M erzeugt (Figur 4). Das Lichtmuster M kann beispielsweise ein Kreisring sein, wie es in Figur 4 gezeigt ist, oder alternativ auch nur wenigstens ein Abschnitt davon, also wenigstens ein Kreisringbogen.

Die Projektionsfläche 32 ist beispielsgemäß eine ebene Fläche. Beim Ausführungsbeispiel ist die Projektionsfläche 32 an einem Schirm 33 der Prüfvorrichtung vorhanden. Die Projektionsfläche 32 kann beispielsweise rechtwinklig zur Erstreckungsrichtung der Elektrode 13 angeordnet sein und nahe am freien Ende der Elektrode 13 oder mit Kontakt dazu angeordnet sein.

Auf der der Projektionsfläche 32 entgegengesetzten Rückseite 34 des Schirms 33 ist optional eine Kamera 35 der Prüfvorrichtung 30 angeordnet. Der Schirm 33 ist für das Licht der Lichtquelle 31 zumindest teilweise transparent, sodass die Kamera 35 das auf der Projektionsfläche 32 erzeugte Lichtmuster M auf der Rückseite 34 erfassen kann. Die Kamera 35 ist mit einer Zentraleinheit 36 kommunikationsverbunden. Ein von der Kamera 35 aufgenommenes Bild des Lichtmusters M kann an die Zentraleinheit 36 übermittelt werden. Die Zentraleinheit 36 kann dazu eingerichtet sein, die Lichtquelle 31 und die Kamera 35 zu steuern. Mittels einer Halterung 37 der Prüfvorrichtung 30 wird beispielsgemäß das Griffstück 14 gegenüber der Projektionsfläche 32 in eine definierte Position und/oder Ausrichtung gebracht. Über die Halterung 37 wird somit auch die Relativposition und Relativausrichtung der distalen Stirnfläche 24 des Lichtwellenleiters 20 gegenüber der Projektionsfläche 32 definiert.

Der Lichtwellenleiter 20 ist beim Ausführungsbeispiel eine Multimodenfaser (MMF). Die proximale Stirnfläche 23 und die distale Stirnfläche 24 sind beispielsgemäß als ebene Flächen ausgeführt und könnten in Abwandlung hierzu auch konvex gekrümmt sein.

Die Lichtquelle 31 kann beispielsweise eine Halbleiterlichtquelle sein und Licht mittels einer Leuchtdiode oder Laserdiode erzeugten.

In einem Prüfzustand, der zur Unterscheidung gegenüber einem optional weiteren Prüfzustand als erster Prüfzustand bezeichnet wird, wird das Licht der Lichtquelle 31 am proximalen Ende 21 in den Lichtwellenleiter 22 unsymmetrisch und/oder exzentrisch gegenüber einem Mittelpunkt P der proximalen Stirnfläche 23 eingekoppelt, wie es stark schematisiert in den Figuren 2 und 3 zu erkennen ist. Bei einem hier beschriebenen Ausführungsbeispiel wird das Licht der Lichtquelle 31 in einem Flächenbereich A der proximalen Stirnfläche 23 eingekoppelt, der beispielsweise eine kreisrunde oder elliptische Form aufweisen kann. Der Flächenbereich A hat einen Flächenschwerpunkt S. Der Flächenschwerpunkt S ist mit Abstand zum Mittelpunkt P der proximalen Stirnfläche 23 angeordnet, wie es in Figur 3 schematisch veranschaulicht ist. Alternativ zu der Darstellung in Figur 3 kann der Mittelpunkt P auch innerhalb des Flächenbereichs A angeordnet sein, in den Licht der Lichtquelle 31 eingekoppelt wird. In jedem Fall ist der Flächenbereich A, in den Licht der Lichtquelle 31 eingekoppelt wird, so gewählt, dass Licht der Lichtquelle 31 radial zum Mittelpunkt P und/oder in Umfangsrichtung um den Mittelpunkt P ungleichmäßig in die proximale Stirnfläche 23 eingekoppelt wird.

In Abwandlung zu dem in Figur 3 gezeigten Beispiel kann der Flächenabschnitt A unzusammenhängend sein, sodass Licht der Lichtquelle 31 in mehrere voneinander beabstandete Abschnitte des Flächenbereichs A in die proximale Stirnfläche 23 eingekoppelt werden kann.

Wie es in Figur 2 schematisch veranschaulicht ist, kann zu der Prüfvorrichtung 30 optional eine Kopplungseinrichtung 38 gehören, die zur optischen Kopplung des Lichtwellenleiters 20 mit der Lichtquelle 31 eingerichtet ist. Die Kopplungseinrichtung 38 kann beispielsweise dazu eingerichtet sein, eine optische Verbindung zwischen dem Verbindungselement 16 und der Lichtquelle 31 derart herzustellen, dass das Licht der Lichtquelle 31 - wie vorstehend beschrieben - unsymmetrisch bzw. exzentrisch gegenüber dem Mittelpunkt P der proximalen Stirnfläche 23 eingekoppelt wird.

Um eine Modemischung während der Prüfung im ersten Prüfzustand zu vermeiden, ist der Lichtwellenleiter 20 während der Prüfung vorzugsweise ausreichend gerade ausgerichtet und kann sich zwischen dem proximalen Ende 21 und dem distalen Ende 22 im Wesentlichen geradlinig erstrecken, wie es schematisch in Figur 2 veranschaulicht ist. Abweichend hierzu könnte der Lichtwellenleiter 20 auch in kleineren Bögen mit ausreichen geringer Krümmung verlaufen, um eine Modemischung zu vermeiden.

Durch die im ersten Prüfzustand unsymmetrische und/oder exzentrische Einkopplung von Licht der Lichtquelle 31 in die proximale Stirnfläche 23 erzeugt das an der distalen Stirnfläche 24 austretende Licht auf der Projektionsfläche 32 das Lichtmuster M, beispielsgemäß ein Lichtmuster M in Form eines geschlossenen Kreisrings mit einem Außenradius ra und einem Innenradius ri (Figur 4).

Anhand der Form des Lichtmusters M und/oder wenigstens einer Dimension des Lichtmusters M in wenigstens einer Raumrichtung parallel zur Projektionsfläche 32 kann im ersten Prüfzustand geprüft werden, ob die Relativpositionen relativer Ausrichtung des distalen Endes 22 bzw. der distalen Stirnfläche 24 einerseits relativ zur Elektrode 13 und/oder dem Griffstück 14 andererseits den Vorgaben entspricht. Beispielsweise kann geprüft werden, ob die Istform des Lichtmusters M einer Sollform des Lichtmusters M entspricht, also beispielsweise ob das Lichtmuster M eine Kreisringform oder eine Kreisbogenform aufweist. Zusätzlich oder alternativ können eine oder mehrere Parameter geprüft werden, wie beispielweise ein Istwert für den Innenradius ri und/oder ein Istwert für den Außenradius ra, die jeweils mit einem zugeordneten Sollwert verglichen werden können.

Unter Anwendung wenigstens eines Prüfkriteriums kann im ersten Prüfzustand erkannt werden, ob das medizinische Instrument 11 und insbesondere die Befestigung des Lichtquellenleiters 20 am Griffstück 14 den Vorgaben ausreichend gut entspricht und das medizinische Instrument 11 daher die Qualitätsanforderungen erfüllt.

Der erste Prüfzustand (Figuren 2 bis 4) kann der einzige Prüfzustand des Verfahrens und/oder der Prüfvorrichtung 30 sein. Optional kann ein weiterer, zweiter Prüfzustand des Verfahrens und/oder der Prüfvorrichtung 30 vorhanden sein (Figuren 5 und 6), wobei zwischen dem ersten Prüfzustand und dem zweiten Prüfzustand umgeschaltet werden kann. Dieser optionale zweite Prüfzustand dient dazu, den Sitz der Elektrode 13 im Griffstück 14 bzw. die Relativposition zwischen der Elektrode 13 und dem Griffstück 14 zu prüfen. Dazu wird am distalen Ende 22 des Lichtwellenleiters Licht derart abgestrahlt, dass sich die Elektrode 13 im Lichtweg zwischen dem aus der distalen Stirnfläche 24 austretenden Licht und der Projektionsfläche 32 befindet. Dadurch ist auf der Projektionsfläche 32 einen Schattenbild B der Elektrode zu erkennen (Figur 6). Die Lage des Schattenbilds B relativ zum Lichtmuster M auf der Projektionsfläche 32 kann ausgewertet werden, um die Relativposition zwischen der Elektrode 13 einerseits und dem Griffstück 14 und dem Lichtwellenleiter 20 andererseits zu prüfen. Es kann somit geprüft werden, ob die Elektrode 13 korrekt am Griffstück 14 angeordnet ist.

Im zweiten Prüfzustand wird auf der Projektionsfläche 32 beispielsgemäß ein zusammenhängendes flächiges Lichtmuster M erzeugt (Figur 6). Ohne Berücksichtigung des Schattenbildes B der Elektrode 13 bildet das Lichtmuster M im zweiten Prüfzustand beispielsweise eine vollständig ausgeleuchtete kreisförmige Lichtfläche auf der Projektionsfläche 32. Im zweiten Prüfzustand kann das Licht der Lichtquelle 31 symmetrisch und/oder konzentrisch und/oder in Form eines vollständig ausgeleuchteten, zusammenhängenden - vorzugsweise kreisförmigen - Lichtflecks relativ zum Mittelpunkt P der proximalen Stirnfläche 23 an der proximalen Stirnfläche 23 des Lichtwellenleiters 20 eingekoppelt werden. Zusätzlich oder alternativ kann der Lichtwellenleiter 20 im zweiten Prüfzustand zwischen seinem distalen Ende 22 und seinem proximalen Ende 21 derart angeordnet sein, dass eine Modenmischung auftritt. Beispielsweise kann der Lichtwellenleiter 20 im zweiten Prüfzustand von seiner gestreckten Lage abweichend ausreichend stark mindestens einmal gekrümmt oder gebogen angeordnet werden.

Die Prüfung im ersten und/oder zweiten Prüfzustand kann beispielsweise bei neu hergestellten medizinischen Instrumenten 11 stichprobenartig oder für alle hergestellten medizinischen Instrumente 11 durchgeführt werden. Zusätzlich besteht auch die Möglichkeit, ein medizinisches Instrument 11 wiederholt nach dessen Einsatz und/oder Sterilisation zu prüfen, sodass erkannt werden kann, wenn aufgrund von äußeren Einwirkungen, insbesondere Temperatureinwirkungen beim Sterilisieren, Veränderungen wie plastische Verformungen oder dergleichen auftreten, die dazu führen, dass das medizinische Instrument 11 nicht mehr den Anforderungen entspricht. Beispielsweise kann durch Temperatureinflüsse die Klebeverbindung zwischen dem distalen Ende 22 des Lichtwellenleiters 20 und dem Griffstück 14 beeinträchtigt werden, wodurch sich die Ausrichtung oder Position des distalen Endes 22 relativ zum Griffstück 14 ändern kann.

Die Erfindung betrifft ein Verfahren und eine Prüfvorrichtung 30 um zu prüfen, ob die Relativposition und Relativausrichtung eines distalen Endes 22 eines Lichtwellenleiters 20 relativ zu einer Elektrode 13 und/oder einem Griffstück 14 eines medizinischen Instruments 11 den Vorgaben entspricht und somit korrekt ist. Dazu wird Licht in ein proximales Ende 21 des Lichtwellenleiters 20 eingekoppelt und am distalen Ende 22 des Lichtwellenleiters 20 auf eine Projektionsfläche 32 zumindest teilweise emittiert. Am proximalen Ende 21 wird das Licht der Lichtquelle 31 exzentrisch und/oder unsymmetrisch gegenüber einem Mittelpunkt P einer proximalen Stirnfläche 23 des Lichtwellenleiters 20 eingekoppelt. Dadurch entsteht auf der Projektionsfläche 32 ein Lichtmuster M, insbesondere ein ringförmiges Lichtmuster M, das anhand von wenigstens einem Prüfkriterium geprüft werden kann, um die korrekte Anordnung bzw. Ausrichtung des distalen Endes 22 zu prüfen. Die Prüfung kann automatisch mittels einer Kamera 35 und einer Zentraleinheit 36 erfolgen, oder durch eine Bedienperson durch Beobachtung des Lichtmusters M auf der Projektionsfläche 32 durchgeführt werden. Optional kann in einem weiteren Prüfzustand geprüft werden, ob die Elektrode 13 korrekt am Griffstück 14 angeordnet ist.

### Bezugszeichenliste:

- 10: System
- 11: medizinisches Instrument
- 12: Gerät
- 13: Elektrode
- 14: Griffstück
- 15: Kabel
- 16: Verbindungselement
- 17: Anschluss
- 18: Kern
- 19: Ummantelung
- 20: Lichtwellenleiter
- 21: proximales Ende
- 22: distales Ende
- 23: proximale Stirnfläche
- 24: distale Stirnfläche
- 25: Auswerteeinheit
- 26: Gewebe

- 30: Prüfvorrichtung
- 31: Lichtquelle
- 32: Projektionsfläche
- 33: Schirm
- 34: Rückseite
- 35: Kamera
- 36: Zentraleinheit
- 37: Halterung
- 38: Kopplungseinrichtung

- A: Flächenbereich
- B: Schattenbild
- L: Licht
- M: Lichtmuster
- P: Mittelpunkt
- ra: Außenradius
- ri: Innenradius
- S: Flächenschwerpunkt

## Patentansprüche

1. Verfahren zur Prüfung einer Ausrichtung und/oder Positionierung eines distalen Endes (22) eines Lichtwellenleiters (20) eines medizinischen Instruments (11) relativ zu einem Griffstück (14) des medizinischen Instruments (11) und/oder relativ zu einer Elektrode (13) des medizinischen Instruments (11), umfassend folgende Schritte:
- Anordnen einer distalen Stirnfläche (24) des distalen Endes (22) des Lichtwellenleiters (20) in einer vorgegebenen Ausrichtung relativ zu einer Projektionsfläche (32),
- Einkoppeln von Licht einer Lichtquelle (31) in eine proximale Stirnfläche (23) an einem dem distalen Ende (22) entgegengesetzten proximalen Ende (21) des Lichtwellenleiters (20) unsymmetrisch und/oder exzentrisch relativ zu einem Mittelpunkt (P) der proximalen Stirnfläche (23),
- Abstrahlen zumindest eines Teils des eingekoppelten Lichts von der distalen Stirnfläche (24) am distalen Ende (22) des Lichtwellenleiters (20) auf die Projektionsfläche (32), und
- Auswerten eines auf der Projektionsfläche (32) erzeugten Lichtmusters (M) basierend auf wenigstens einem vorgegebenen Prüfkriterium zur Prüfung der Ausrichtung des distalen Endes (22) des Lichtwellenleiters (20).

2. Verfahren nach Anspruch 1, wobei das wenigstens eine Prüfkriterium das Prüfen einer Abweichung zwischen einer Istform des Lichtmusters (M) auf der Projektionsfläche (32) und einer vorgegebenen Sollform aufweist.

3. Verfahren nach Anspruch 1 oder 2, wobei eine Istform und/oder eine Sollform des Lichtmusters (M) ein geschlossener Ring oder ein Ringbogen ist.

4. Verfahren nach einem der vorhergehenden Ansprüche, wobei das wenigstens eine Prüfkriterium das Prüfen einer Abweichung zwischen einem Istwert einer Dimension des Lichtmusters (M) in wenigstens einer Raumrichtung parallel zur Projektionsfläche (32) und einem vorgegebenen Sollwert der Dimension in dieser Raumrichtung umfasst.

5. Verfahren nach Anspruch 3 und 4, wobei die Abweichung zwischen einem Sollwert und einem Istwert des Innenradius (ri) und/oder des Außenradius (ra) des Rings oder Ringbogens geprüft wird.

6. Verfahren nach einem der vorhergehenden Ansprüche, wobei ein Bild des Lichtmusters (M) auf der Projektionsfläche (32) mittels einer Kamera (35) erfasst und das Bild zur automatischen Auswertung basierend auf dem wenigstens einen Prüfkriterium an eine Zentraleinheit (36) übermittelt wird.

7. Verfahren nach Anspruch 5, wobei die Zentraleinheit (36) dazu eingerichtet ist, die Kamera (35) und die Lichtquelle (31) zu steuern.

8. Verfahren nach einem der vorhergehenden Ansprüche, wobei der Lichtwellenleiter (20) eine Multimodenfaser ist.

9. Verfahren nach Anspruch 7, wobei der Lichtwellenleiter (20) zwischen seinem distalen Ende (22) und seinem proximalen Ende (21) derart angeordnet wird, dass eine Modenmischung vermieden wird.

10. Verfahren nach Anspruch 8, wobei der Lichtwellenleiter (20) zwischen seinem distalen Ende (22) und seinem proximalen Ende (21) im Wesentlichen gestreckt ist.

11. Verfahren nach einem der vorhergehenden Ansprüche, wobei das Licht der Lichtquelle (31) in einen Flächenbereich (A) der proximalen Stirnfläche (23) des Lichtwellenleiters (20) eingekoppelt wird, wobei der Flächenschwerpunkt (S) des Flächenbereichs (A) versetzt ist zum Mittelpunkt (P) der proximalen Stirnfläche (23).

12. Verfahren nach einem der vorhergehenden Ansprüche, wobei die proximale Stirnfläche (23) und/oder die distale Stirnfläche (24) eine ebene Fläche ist.

13. Verfahren nach einem der vorhergehenden Ansprüche, wobei in einem anderen Prüfzustand die Elektrode (13) von an der distalen Stirnfläche (24) austretendem Licht beleuchtet wird, um ein Schattenbild (B) der Elektrode (13) auf der Projektionsfläche (32) zu erzeugen und anhand des Schattenbildes (B) zu prüfen, ob die Elektrode (13) relativ zum Griffstück (14) korrekt angeordnet ist.

14. Prüfvorrichtung (30) zur Durchführung des Verfahrens nach einem der vorhergehenden Ansprüche, aufweisend:
- eine Lichtquelle (31) zum unsymmetrischen und/oder exzentrischen Einkopplung von Licht in eine proximale Stirnfläche (23) an einem proximalen Ende (21) eines Lichtwellenleiters (20) relativ zu dessen Mittelpunkt (P),
- eine Projektionsfläche (32) und
- eine Halterung (37) zur Anordnen eines distalen Endes (22) des Lichtwellenleiters (20) in einer vorgegebenen Relativposition relativ zur Projektionsfläche (32).

15. Prüfvorrichtung nach Anspruch 14, außerdem aufweisend eine Kamera (35) zur Aufnahme eines Bildes eines auf der Projektionsfläche (32) erzeugten Lichtmusters (M).

## Claims

1. Method for testing an orientation and/or positioning of a distal end (22) of an optical fiber (20) of a medical instrument (11) relative to a handle piece (14) of the medical instrument (11) and/or relative to an electrode (13) of the medical instrument (11), comprising the following steps:
- arranging a distal face (24) of the distal end (22) of the optical fiber (20) in a predefined orientation relative to a projection surface (32),
- coupling light of a light source (31) into a proximal face (23) at a proximal end (21) of the optical fiber (20) opposite the distal end (22) in an unsymmetric and/or eccentric manner relative to a center (P) of the proximal face (23),
- emitting at least a part of the coupled light from the distal face (24) at the distal end (22) of the optical fiber (20) onto the projection surface (32), and
- evaluating a light pattern (M) created on the projection surface (32) based on at least one predefined test criterion for testing the orientation of the distal end (22) of the optical fiber (20).

2. Method according to claim 1, wherein the at least one test criterion comprises the test of a deviation between an actual shape of the light pattern (M) on the projection surface (32) and a predefined desired shape.

3. Method according to claim 1 or 2, wherein the actual shape and/or the desired shape of the light pattern (M) is a closed ring or ring arc.

4. Method according to any of the preceding claims, wherein the at least one test criterion comprises the test of a deviation between an actual value of a dimension of the light pattern (M) in at least one spatial direction parallel to the projection surface (32) and a predefined desired value of the dimension in this spatial direction.

5. Method according to claims 3 and 4, wherein the deviation between a desired value and an actual value of the inner radius (ri) and/or the outer radius (ra) of the ring or ring arc is tested.

6. Method according to any of the preceding claims, wherein an image of the light pattern (M) on the projection surface (32) is captured by means of a camera (35) and the image is transmitted to a central unit (36) for automatic evaluation based on the at least one test criterion.

7. Method according to claim 5, wherein the central unit (36) is configured to control the camera (35) and the light source (31).

8. Method according to any of the preceding claims, wherein the optical fiber (20) is a multi-mode fiber.

9. Method according to claim 7, wherein the optical fiber (20) is arranged between its distal end (22) and its proximal end (21) so that mode mixing is avoided.

10. Method according to claim 8, wherein the optical fiber (20) is substantially stretched between its distal end (22) and its proximal end (21).

11. Method according to any of the preceding claims, wherein the light of the light source (31) is coupled into a surface section (A) of the proximal face (23) of the optical fiber (20), wherein the centroid of an area (S) of the surface section (A) is offset relative to the center (P) of the proximal face (23).

12. Method according to any of the preceding claims, wherein the proximal face (23) and/or the distal face (24) is a planar surface.

13. Method according to any of the preceding claims, wherein in another test condition the electrode (13) is illuminated by light exiting the distal face (24) in order to create a silhouette (B) of the electrode (13) on the projection surface (32) and in order to test based on the silhouette (B) whether the electrode (13) is correctly arranged relative to the handle piece (14).

14. Test device (30) for carrying out the method according to any of the preceding claims comprising:
- a light source (31) for coupling light into a proximal face (23) at a proximal end (21) of an optical fiber (20) unsymmetrically and/or eccentrically relative to its center (P),
- a projection surface (32) and
- a holder (37) for arranging a distal end (22) of the optical fiber (20) in a predefined relative position relative to the projection surface (32).

15. Test device according to claim 14, further comprising a camera (35) for capturing an image of a light pattern (M) created on the projection surface (32).

## Revendications

1. Procédé de contrôle d'une orientation et/ou du positionnement d'une extrémité distale (22) d'un guide d'ondes optiques (20) d'un instrument médical (11) par rapport à un manche (14) de l'instrument médical (11) et/ou par rapport à une électrode (13) de l'instrument médical (11), comprenant les étapes suivantes :
- mise en place d'une face frontale distale (24) de l'extrémité distale (22) du guide d'ondes optiques (20) avec une orientation prédéfinie par rapport à une surface de projection (32),
- injection de la lumière d'une source lumineuse (31) dans une face frontale proximale (23) sur une extrémité proximale (21) du guide d'ondes optiques (20) opposée à l'extrémité distale (22), de manière asymétrique et/ou excentrique par rapport à un centre (P) de la face frontale proximale (23),
- rayonnement d'au moins une partie de la lumière injectée, à partir de la face frontale distale (24) à l'extrémité distale (22) du guide d'ondes optiques (20), sur la surface de projection (32), et
- évaluation d'un motif lumineux (M) généré sur la surface de projection (32), sur la base d'au moins un critère de contrôle prédéfini, aux fins de contrôle de l'orientation de l'extrémité distale (22) du guide d'ondes optiques (20).

2. Procédé selon la revendication 1, selon lequel le critère de contrôle, au nombre d'au moins un, comprend le contrôle d'un écart entre une forme réelle du motif lumineux (M) sur la surface de projection (32) et une forme de consigne prédéfinie.

3. Procédé selon la revendication 1 ou 2, selon lequel une forme réelle et/ou une forme de consigne du motif lumineux (M) est un anneau fermé ou un arc d'anneau.

4. Procédé selon une des revendications précédentes, selon lequel le critère de contrôle, au nombre d'au moins un, comprend le contrôle d'un écart entre une valeur réelle d'une dimension du motif lumineux (M), dans au moins une direction spatiale parallèle à la surface de projection (32), et une valeur de consigne prédéfinie de la dimension dans cette direction spatiale.

5. Procédé selon la revendication 3 ou 4, selon lequel on contrôle l'écart entre une valeur de consigne et une valeur réelle du rayon intérieur (ri) et/ou du rayon extérieur (ra) de l'anneau ou de l'arc d'anneau.

6. Procédé selon une des revendications précédentes, selon lequel une image du motif lumineux (M) sur la surface de projection (32) est recueillie à l'aide d'une caméra (35), et l'image est transmise à une unité centrale (36) en vue d'une évaluation automatique sur la base du critère de contrôle, au nombre d'au moins un.

7. Procédé selon la revendication 5, selon lequel l'unité centrale (36) est conçue pour commander la caméra (35) et la source lumineuse (31).

8. Procédé selon une des revendications précédentes, selon lequel le guide d'ondes optiques (20) est une fibre multimodale.

9. Procédé selon la revendication 7, selon lequel le guide d'ondes optiques (20) est disposé, entre son extrémité distale (22) et son extrémité proximale (21), de manière à ce qu'un mélange de modes soit évité.

10. Procédé selon la revendication 8, selon lequel le guide d'ondes optiques (20) est sensiblement tendu entre son extrémité distale (22) et son extrémité proximale (21).

11. Procédé selon une des revendications précédentes, selon lequel la lumière de la source lumineuse (31) est injectée dans une zone de surface (A) de la face frontale proximale (23) du guide d'ondes optiques (20), le centre de gravité de surface (S) de la zone de surface (A) étant décalé par rapport au centre (P) de la face frontale proximale (23).

12. Procédé selon une des revendications précédentes, selon lequel la face frontale proximale (23) et/ou la face frontale distale (24) est une surface plane.

13. Procédé selon une des revendications précédentes, selon lequel, dans un autre état de contrôle, l'électrode (13) est éclairée par la lumière sortant sur la face frontale distale (24), afin de produire une ombre projetée (B) de l'électrode (13) sur la surface de projection (32) et contrôler à l'aide de l'ombre projetée (B), si l'électrode (13) est disposée correctement par rapport au manche (14).

14. Dispositif de contrôle (30) pour la mise en œuvre du procédé selon une des revendications précédentes, comprenant :
- une source lumineuse (31) pour l'injection asymétrique et/ou excentrique de lumière dans une face frontale proximale (23) à une extrémité proximale (21) d'un guide d'ondes optiques (20), par rapport au centre (P) de celui-ci,
- une surface de projection (32), et
- un support (37) pour la mise en place d'une extrémité distale (22) du guide d'ondes optiques (20) dans une position relative prédéfinie par rapport à la surface de projection (32).

15. Dispositif de contrôle selon la revendication 14, comprenant en outre une caméra (35) destinée à recueillir une image d'un motif lumineux (M) produit sur la surface de projection (32).
